# EUROPEAN PATENT SPECIFICATION

(11) **EP 2 229 936 B1**
(45) Date of publication and mention of the grant of the patent: **06.05.2015**
(21) Application number: 09003370.5
(22) Date of filing: 09.03.2009
(51) Int. Cl.: A61K 9/14, A61K 9/51, A61K 9/107, A61K 9/48, A61K 31/568

(54) **Nanonized testosterone formulations for improved bioavailability**
Nanonisierte Testosteronformulierungen für verbesserte Bioverfügbarkeit
Formules de testostérone de taille nano pour une meilleure biodisponibilité

(43) Date of publication of application: 22.09.2010
(73) Proprietor: PharmaSol GmbH, 12307 Berlin (DE)
(72) Inventor: Keck, Cornelia, 12161 Berlin (DE); Muchow, Marc, 67292 Kirchheimbolanden (DE)
(74) Representative: UEXKÜLL & STOLBERG

(56) References cited:
- EP-A- 1 698 329
- WO-A-00/67728
- WO-A-2007/103294
- DE-A1- 19 825 856
- DE-A1- 19 932 157
- US-A1- 2002 119 916
- US-B1- 6 814 959
- BAGCHUS W M ET AL: "IMPORTANT EFFECT OF FOOD ON THE BIOAVAILABILITY OF ORAL TESTOSTERONE UNDECANOATE" PHARMACOTHERAPY, BOSTON, US, vol. 23, no. 3, 1 January 2003 (2003-01-01), pages 319-325, XP001147255 ISSN: 0277-0008

## Description

### State of the Art:

In general products for testosterone delivery can be divided into two groups: Products for injection and other products which include oral, buccal, and transdermal systems.

The crucial problem is the very high first pass effect of natural testosterone taken orally, about 99% of the drug are immediately metabolised by liver enzymes and do not reach the circulation.

To bypass this problem several approaches have been made: The first attempt was the methylisation of testosterone, which hindered liver enzymes from inactivating it. Although application of methyltestosterone lead to a pronounced serum testosterone level, it was found to be very liver toxic and was therefore withdrawn (C. Wang et al., Investigation, treatment and monitoring of late-onset hypogonadism in males, Int. J. Androl. 32, 1-10 (2008); F. C. Wu, Steroidogenesis and androgen use and abuse, Bailliere's Clin. Endocrinol. Metab. 6, 403-737 (1992)). However, other testosterone esters are better tolerated. There is a variety of testosterone delivery systems on the market consisting of testosterone or its esters, however they all have clear disadvantages.

The first group are injections. In general injections are not patient-convenient. Intravenous injection is performed with an aqueous suspension of testosterone. Due to rapid degradation of the molecule by enzymes, several injections per day are necessary to maintain a sufficiently high serum level of the hormone. As a result the products have been discontinued (Androlan Aqueosus; Andesterone Suspension, both USA) Roughly half a dozen testosterone preparations for interamuscular injection are on the market in the U.S.A., France and Germany. Those products consist of an oily solution of a testosterone ester (mostly testosterone enantate or testosterone undecanoate). After intramuscular injection the hormone is liberated from the oil during a period of 6 to 12 weeks. However, more than 10% of the patients suffer from pain at the site of injection. Even more critical, once administered, the dose cannot be adapted which carries the risk of overdosing - especially with patients that start with a testosterone replacement therapy (M. A. Mackey, Tolerability of intramuscular injections of testosterone ester, in oil vehicle. Hum. Reprod. 10:862-865 (1995)).

Transdermal systems available for application onto the skin are gels and patches. They can be classified by their site of application: Systems which are applied on the torso and systems for the scrotum. Transdermal gels are preparations of testosterone and sometimes contain also permeation enhancers like cyclopentadecanolide or high concentrations of alcohol. They are applied at least once a day and they need to dry on the skin. The patient has to wait for 5 to 10 minutes after application for the formulation to dry. Hands have to be carefully washed and the site of administration has to be covered to avoid other persons (especially children) to get into contact with the gel. If skin contact with untreated persons is expected, showering beforehand is strongly advised. Otherwise it bears the risk of transferring the drug to them and can result (in case of females) in androgenisation. Side effects are skin irritations. Due to inter- and intraindividual differences in skin permeation the flux of drug can greatly vary and is almost impossible to predict. Transdermal patches are also widely used and available on the markets worldwide. Depending on the delivery system, one or even two patches have to by applied simultaneously and have to be carried for two to four days. Severe skin irritations are a common phenomenon. The systems for use on the torso are still available while the formulations for application on the scrotum have been discontinued: While absorption of testosterone via scrotum skin is better, it contains high concentration of esterases that lead to a high concentration of dihydrotestosterone after application. Furthermore compliance was low: Shaving of the scrotum was necessary, problems with adhesion of the patches and skin irritations occurred (J. C. Findlay et al., Treatment of primary hypogonadism in men by the transdermal administration of testosterone, J. Clin. Endocrinol. Metab. (68) 369-373 (1989)). While the problem of high dihydrotestosterone levels does not occur with non-scrotum patches, skin irritation is even more pronounced with these formulations. In general it can be concluded that transdermal systems exhibit delivery problems and are not very patient convenient.

The use of subcutaneous implants is also theoretically possible. There is only one subcutaneous product commercially available, which is only sold in the U.S.A.: Testopel pellets. They are implanted under the skin and release the drug continuously over a four to six months time period. A microsurgery has to be performed to put the 3.4x9mm pellets into their desired place of action. Furthermore once administered the release rate of testosterone is difficult to adjust. Risks and side effects are: Extrusion, bleeding, and inflammations/infections at the site of implantation (F. Jockenhovel et al., Pharmacokinetics and pharmacodynamics of subcutaneous testosterone implants in hypogonadal men. Clin. Endocrinol. (Oxf.) 45:61-71 (1996)). Apart from the implantation procedure being not patient-friendly, it is difficult to terminate a treatment. The low number of these products on the market clearly reflects the little suitablility of this delivery approach.

Buccal administration of testosterone is achieved by the use of a tablet (bioadhesive delivery system), which has to be placed on the gum. A buccal formulation (Striant) is currently marketed in the U.S., Great Britain and The Netherlands. Sufficiently high serum levels can only be achieved if the product is constantly applied and changed every 12 hours. (H. Zhang et al., oral mucosal drug delivery-clinical pharmacokinetics and therapeutic applications. Clin. Pharmacokinet. (41) 661-680 (2002)). The buccal tablet does not always stay in place. Tooth brushing, eating and drinking often loosens it. It is also unintentionally swallowed without even noticing it. Side effects that have been described are: Headache, gingivitis and irritations of the gum and dysgeusia (taste perversion). Regular gum examination is strongly advised (Striant patient information, http://www.striant.com/Consumer/patient_info/patient_info.asp, accessed 04/03/09).

There are a variety of other formulation attempts like nasal spray, pulmonary delivery or ultrasound enhanced transdermal transport. Those attempts have not made it to the market yet.

After revieing the disadvabntages of the various delivery routes, the oral route has clear advantages. Is is patient-convenient with no side effects such as injection pain. In contrast to i.m. injections, treatment can be terminated.

However, as outlined above, the first pass effect limits the amount of testosterone reaching the circulation dramatically. To overcome this problem, capsules filled with a solution of testosterone undecanoate in an oily solution have been developed (e.g. Andriol Testocaps, Essex Pharma GmbH, Germany). The oil is partly taken up by the lymphatic system bypassing the liver and hence the first pass effect. Still, the bioavailability is quite low (around 3%) and therefore a total of up to four capsules have to be taken by the patient with one to four administrations per day. In addition, the testosterone serum level resulting is irregular with peaks of short duration. Another problem of this formulation are the high inter- and intra-individual differences in pharmacokinetic parameters of the product (H. M. Behre and E. Nieschlag, Comparative pharmacokinetics of testosterone esters, in: E. Nieschlag and H. M. Behre (eds.), Testosterone-Action, Deficiency, Substitution, 2nd ed., Springer- Verlag, Berlin, pp. 329-348 (1998)).

Since the major part of the oral dose is not taken up lymphatically but takes the classic route via the liver, the stress on this organ is very high. Furthermore the amount of capsules to be taken every day and the dosing scheme does not foster compliance (Nieschlag (2008), Testosterone treatment comes of age: new options for hypogonadal men, Clinical Endocrinology, Volume 65, Issue 3, Pages 275-281; Bagchus et al. (2003), Important effect of food on the bioavailability of oral testosterone undecanoate, Pharmacotherapy, 23, 319-325); D.M. Shackleford et al. (2003), The contribution of lymphatically transported testosterone undecanoate to the systemic exposure of testosterone after oral administration of two Andriol formulations in conscious lymph-duct cannulated dogs, J. Pharmacol. Exp. Ther., 306, 925-933).

To summarize, oral treatment with testosterone has clear advantages compared to the other administration routes. However, the presently available formulation needs clear improvement. The bioavailability (BA) should be higher, to be able to reduce the number of capsules or tablets to be taken as a single dose (normally 2 for Andriol Testocaps). Ideally the doses administered should be lower. This would reduce the stress on the liver caused by the drug not lymphatically absorbed but passing the liver. This can also be achieved when having an oral formulation with higher bioavailability. This invention provides a nanonized formulation which fulfils this. The formulation can be used for testosterone derivatives, e.g. esters, but also for the original testosterone molecule. In addition the nanonized formulations can be potentially applied for dermal delivery or nasal administration.

### Aim of invention:

Aim of the invention was to reduce the single dose of testosteronundecanoate (TU) from 2 units (capsules) - as used in the commercial product Andriol Testocaps^{®} - to one unit by replacing the oil formulation of TU by one which shows identical - or ideally higher - bioavalibility (BA) but requires less application volume. A single dose improves patient compliance, reduces costs of the health system caused by non-compliance and increases the convenience of the patient in therapy. Ideally, the formulation should not only allow for delivering the synthetic derivative TU, but also the original natural biological molecule testosterone (T). Instead of T, TU is on the market, because the bioavailability of T is even lower (i.e. even more oral units per single dose would be required).

### Short description of the invention:

The BA of TU and T were increased by nanonizing the drugs. Nanonizing was performed either by incorporating the drugs into lipid nanoparticles (nano lipid carriers - NLC), or alternatively by just reducing the size of the drug without the presence of a lipid.

In case of lipid nanoparticles, TU was incorporated in a matrix from a lipid being solid at body temperature. The TU dissolved in oil was added to the melt of the solid lipid (e.g. stearic acid), mixed and then the drug containing lipid melt was dispersed in a hot surfactant solution to yield a coarse pre-emulsion. This coarse pre-emulsion was subsequently homogenized at elevated temperature using a Micron LAB 40 (APV Deutschland GmbH, Germany), typically applying 500 bar and various cycles depending on the size to be achieved. Basically the production process for NLC was employed as described in PCT application no. PCT/EP2000/004112 and in United States Patent 6814959.

In addition TU and T were nanonized in the absence of a lipid particle matrix. TU or T were dispersed in a surfactant solution and then homogenized using the same homogenizer, typically at 1,500 bar and up to 20 cycles. Basically the process was employed described in PCT application no. PCT/EP2000/006535 but using in this case an aqueous surfactant solution as dispersion medium. However, also other dispersion media are suitable.

In principle all size diminution processes can be used for generating the nanonized TU and T particles. Examples are pearl milling, and combination processes of a pre-treatment step with high pressure homogenization (HPH), e.g. pearl milling followed by HPH (so called CT process, PCT application no. PCT/EP2007/009943).

From the literature and the studies performed within the screening, it,is established that the bioavailibilty (BA) of TU increases with the presence of lipid. Administering the marketed product Andriol Testocaps^{®} with a higher amount of oil increased the BA (example 1). In contrast to this, it was surprisingly found, that even at lower lipid concentrations than contained in the Andriol Testocaps^{®} oil capsule, a higher BA could be achieved when administering TU in the form of lipid nanoparticles. The same higher BA was achieved with NLC, even when lowering the lipid:drug ratio (example 4).

The BA increased with decreasing size of the lipid nanoparticles. The BA was dependent to a limited extent on the nature of the lipid particle matrix, glycerides yield higher BA than e.g. fatty acids. The lipid overall content was generally lower compared to Andriol, and even high BAs were obtained at lower lipid:drug ratios (in contrast to Andriol, where more lipid is needed to increase the BA).

A relatively high BA was also achieved when producing TU and T particles in the nanometer range without having any lipid present. This is against the teaching and state of the art in the literature, suggesting that only with the lipid a sufficiently high BA can be reached.

Using either the lipid based nanonized TU and T or the nanonized TU or T without lipid yields BAs sufficiently high to produce one oral unit (tablet, capsule) for delivering the single dose. The 2 units of Andriol Testocaps^{®} can be replaced by one unit for improvement of therapy by increased patient compliance and patient convenience.

The nanonized TU and T formulations can also be used for routes of administration other than the oral route. They can be administered dermally, by the nasal route as spray or gel, pulmonary by nebulisation as aerosol, intravenously by injection, and other routes of injection, e.g. intramuscularly as depot.

### Detailed description of the invention:

A dose of 2 capsules with a total amount of oil solution of about 670mg is required due to the low solubility of TU in oils. To dissolve the single dose of 2*40 mg, this oil mass is necessary. It is known that Andriol Testocaps^{®} show a lower bioavailibility when taken in the non-fed state, the bioavailability increases in the fed state. That means to increase the bioavailability, it would be advantageous to administer the single dose with even more oil compared to the marketed capsule. The effect of increasing oil is shown in example 1. TU taken from the product Andriol Testocaps^{®} and dispersed in oil (4 mg TU dispersed in oil yielding a total weight of 400mg) shows a distinctly higher BA (13,105 units, 1 unit = 1pg*h/ml) compared to the same amount of TU taken from Andriol Testocaps^{®} (4 mg TU in oil, total weight: 33,4mg, taken from Andriol Testocaps^{®} capsule) but dispersed in water (BA 8,542 units). Based on these data and in accordance with the literature, it would be logic to administer TU in an even larger oil volume than in the product Andriol Testocaps^{®} to achieve a higher bioavailibility.

In contrast to this, it was found that the administration of the same amount of TU incorporated in a lower amount of lipid showed higher BA than Andriol Testocaps^{®} (administered dispersed in surfactant solution). TU was incorporated in lipid nanoparticles, precisely nanostructured lipid carriers - NLC (NLC suspension with 10% particles in water, administered was an amount of 133mg containing 13,3 mg particles, composed of 9,3 mg lipid and 4mg TU, that means the lipid:drug ratio was 70:30). (example 2). The study further shows that the BA increases with decreasing particles size, being about10,200 units for the 600nm particles, and about 14,000 units for the 200 nm particles (reference: 8,542 units for Andriol Testocaps^{®}).

In another study, the effect of using different solid lipids was investigated by incorporating TU in lipid nanoparticles containing either stearic acid or stearic acid triglyceride (Dynasan 118). In this case the lipid particle suspensions also contained 10% particles (the ratio lipid:TU was 85:15, that means 26.6mg particles composed of 4mg TU and 22.6g lipid were administered). The BAs were similar for both solid lipids, being around 12,000 - 13,000 units (example 3). That means there was limited effect of the nature of the lipid on the BA, but the BA was slightly higher for Dynasan 118. Both lipids are suitable carrier materials.

In another study, two 200 nm lipid nanoparticle formulations were compared. They differed only in the lipid:drug ratio which was 70:30 (3.0% TU NLC suspension) and 85:15 (1.5% TU NLC suspension) Both were delivered in a 10% particle suspension and had the same amount of TU per administration (4mg). From example 4 it can be concluded:
In contrast to the teaching in the state of art, smaller amounts of lipids in form of lipid nanoparticles result in similar or even a higher BA. The formulation with less lipid gives better BA than the formulation with higher lipid:drug ratio.

As results from these and other studies it could be concluded:
1. A higher BA compared to Andriol Testocaps^{®} can be achieved when administering TU nanonized, that means in the form of lipid nanoparticles.
2. To achieve this higher BA less amount of lipid is necessary compared to the marketed product Andriol Testocaps^{®}.
3. This allows to incorporate the single dose of TU in one oral unit (e.g. tablet or capsule).
4. The increase in BA allows reducing the administered dose but achieving the same AUC in the blood as Andriol Testocaps^{®}.
5. The BA increases with decreasing particles size. The particles should be below 1000nm, preferentially below 500 nm, ideally 200 or below, most favourable below 100 nm.
6. The nature of the solid lipid in the lipid nanoparticles has a limited effect, despite triglycerides seem to be slightly better in enhancing the BA compared to fatty acids.

In another study TU was administered in a nanonized form but without adding a lipid. This simple nanonization process has the advantage, that it is technically simpler compared to making lipid nanoparticles. According to the literature describing the strong influence of lipid present on the BA, a very low BA was expected. TU taken from the Andriol Testocaps^{®} capsule yielded a BA of 8,542 units (administered were 4mg TU in 33,4mg oil solution). The nanonized TU without any lipid present reached still a BA of 6,616 units (example 5).

From this is can be concluded, that
1. nanonization is one of the BA enhancing effects
2. with nanonized TU the single dose -identical to TU-loaded lipid nanoparticles - can also be delivered in one single oral unit (e.g. tablet or capsule)
3. adding small amounts of lipid (even very small amounts compared to the Andriol Testocaps^{®} capsule) in form of lipid nanoparticles further enhances the BA.

To compensate for the slightly lower BA, the dose can be slightly increased. Administration in one single unit will still be possible, because 80 mg TU, or even higher doses of TU in one tablet are possible.

In the same study, Testosteron (T) was administered in nanonized form (example 5). The bioavailibilty was 3,850 units. This was about half of the BA of TU, but T is very well known to show an extreme first pass effect by the liver, being the reason that normally testosterone derivatives such as TU are being used. Considering this, the achieved BA with T was still remarkable. That means also for T nanonization proved to be very efficient in increasing the BA. This is normally only expected for the drugs of class II of the biopharmaceutical classification system (BCS, low BA due to poor solubility), but not for drugs showing poor permeability or high liver first pass effect (e.g. drugs of BCS class IV).

The size of the produced and tested particles was determined applying photon correlation spectroscopy (PCS). Measurements were performed using a Zetasizer 2000HS (Malvern Instruments, Malvern, UK) applying a measuring angle of 90° at a constant room temperature. They were repeated 10 times. All samples were diluted with ultrapure water to an appropriate concentration. PCS yields a mean diameter (z-average) and a polydispersity index (PI) as a measure for the width of the size distribution. The sizes given are all PCS data, including the size specifications in the claims.

In vivo studies: Male Wistar rats with a standardized weight of about 400g were used for the in vivo studies. The dose was adjusted to the exact weight of the respective weight of each rat, the figures given in the examples are calculated for a 400g rat. The dose administered each time was 4mg TU or 2.53mg T respectively, corresponding to a dose of 10mg/kg body weight TU. Rats were housed in groups of 4 in cages under standard conditions: room temperature (21 ± 3°C), light/dark cycle (12/12 h with lights on from 7 a.m. to 7 p.m.). They had free access to food and water. The three groups consisted of a minimum of 4 rats each. One additional rat per group was used to possibly replace an animal.

Animals were deprived from food 12 hours prior to sample administration. They had access to water ad libitum during the whole study. For the oral administration of the samples, a feeding needle was used. Blood sampling was done at different times (t=0h, 1h, 2h, 3h, 4h, 6h, and 8h) after administration. Approximately 400 µL of blood were collected using sterile syringes with a needle.

The tubes were then gently mixed and held at 4°C for 30 minutes and then centrifuged at 4°C for 15 min at 6000g. The serum obtained was immediately transferred into 1.5 ml polypropylene vials and stored at -80°C for later analysis.

Serum analysis in the in vivo studies was performed using an enzyme immunoassay (EIA) test No. 582701 from Cayman Chemicals (USA) (Ann Arbor, Michigan, USA). Analysis was performed following the extraction procedure provided by the manufacturer. The test was used exactly as directed. Recovery rates were determined using a cold spike method and found to be approx. 90%. The plate was read using a microplate reader at 412nm wavelength. Results were calculated following the instructions given by the manufacturer using the provided spreadsheet (MS Excel 2003).

The assay test is based on the competition of testosterone and a testosterone tracer for a limited amount of testosterone antiserum. The concentration of the tracer is held constant, the concentration of testosterone varies (depending on the serum level of the sample). Therefore the amount of tracer which can bind to the antiserum is inversely proportional to the testosterone concentration of the sample. Ellman's reagent is an substrate to the tracer and is used to determine the amount of bound tracer. The product of the enzymatic reaction has a yellow colour. Its concentration can be assessed using a photometer (microplate reader).

General use of the particles of the invention: The particles can be incorporated into tablets, buccal tablets and pellets e.g. by transforming them into a powder by spray drying or lyophilisation, and then performing a standard production process. Alternatively, the dispersion can be used as granulation fluid in tablet production or wetting agent in pelletization. Filling of capsules can be performed using e.g. the powder. Other applications are incorporation into polymer films for oral administration but also dermal application. The membrane permeation enhancing effect of the particles can also be exploited for other application routes, e.g. dermal or mucosal. The particles can be incorporated into matrices of dermal, transdermal and mucosal patches, or in gels or creams. The liquid particle dispersions can also be the final dosage form, e.g. as oral suspension, nasal spray or even use in nebulizers for pulmonary delivery.

### Legends of figures:

**Fig. 1****:** BA of TU from Andriol Testocaps^{®} capsule dispersed in surfactant solution (left) and diluted with oil (right) (example 1).
**Fig. 2****:** BA of TU incorporated in 200 nm and in 600 nm NLC compared to Andriol Testocaps^{®} administered dispersed in Tween 80 solution (example 2).
**Fig. 3****:** BA of NLC with similar size of about 200 nm, but made from different lipids (stearic acid, left and Dynasan 118, right) versus BA of Andriol Testocaps^{®} (administered dispersed in surfactant solution).
**Fig. 4****:** BA of NLC with similar size of 200 nm, but different lipid drug ratio (example 4), 15% versus 30% TU in lipid particle matrix of NLC suspension. Increasing the size from 200 nm to 600 nm decreases the BA, but all BAs are still higher compared to the marketed product Andriol Testocaps^{®} (right).
**Fig. 5****:** BA of Andriol Testocaps^{®} marketed product versus BA of nanonized TU and T without addition of lipid.
**Fig. 6****:** BA of 40 nm TU nanoparticles versus BA of Andriol Testocaps^{®}.

### Examples:

### Example 1: BA of TU from Andriol Testocaps^{®} dispersed in oil compared to TU dispersed in surfactant solution.

The amount of 4 mg TU contained in 33.4mg oil solution of the capsule was dispersed in surfactant solution (0.1% Tween 80 in water) yielding a total amount of 400mg, and administered orally to rats. This corresponds to administering Andriol Testocaps^{®} to non-fed patients.

The second test formulation was the same amount of 4mg TU contained in 33.4mg oil solution of the capsule, but this time diluted with the oil of the capsule itself to yield the same amount of 400mg. One capsule Andriol Terstocaps contains 40 mg TU dissolved in an oil solution with a total amount of about 330 mg. Administering this capsule to humans with the same amount of added oil would mean that the patient has to take about 4g pure oil, corresponding to the fed state of absorption.

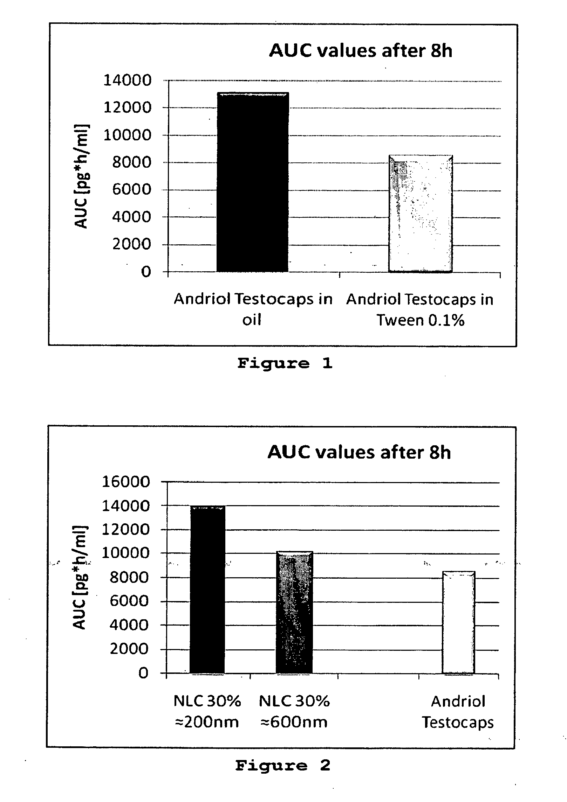

After oral administration the BA was determined as described in the analytical procedure, yielding an area under the curve of 8,542 units for Andriol Testocaps^{®} dispersed in surfactant solution compared to 13,105 units AUC for the TU diluted with oil (Fig. 1). This shows that increasing the amount of lipid enhances the BA.

### Example 2: BA of TU incorporated into lipid nanoparticles of different size versus BA of Andriol Testocaps^{®}.

TU was incorporated into lipid nanoparticles. The concentration of the lipid nanoparticles in the aqueous suspension was 10% (w/w). The composition of the NLC was:

| | |
|---|---|
| Dynasan118 | 3.5% |
| oleic aicd | 3.5% |
| TU | 3.0% |
| Tween 80 | |
| water up to | 100.0% |

The Tween 80 concentration was 2% in case of 200nm, and 1% Tween in case of 600nm NLC.

Different particle sizes of 200 nm and 600 nm were produced by varying the homogenization conditions and the concentration of surfactant. Administered per rat were 133mg NLC suspension. The BA was 10,208 units for the NLC with 600nm, and 13,950 units for the 200 nm NLC (Fig. 2)

### Example 3: Effect of nature of solid lipid on BA of TU: Stearic acid versus Dynasan 118.

NLC suspension with a lipid particle content of 10% were prepared using different solid lipids, that means stearic acid as a fatty acid and Dynasan 118 as a glyceride. The composition of the NLC was (w/w%):

| | |
|---|---|
| solid lipid | 4.25% |
| oleic aicd | 4.25% |
| TU | 1.5% |
| Tween 80 | 2.0% |
| water up to | 100.0% |

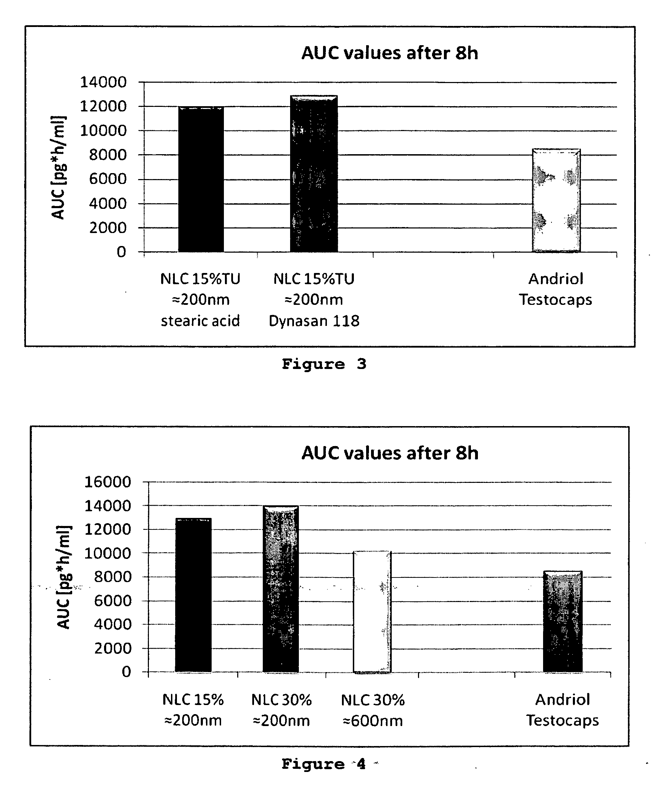

267 mg of the NLC suspensions were administered to rats. The BA of both formulations were similarily high, being 11,976 units for stearic acid and 12,933 for Dynasan 118 (Fig. 3). Obviously there was limited effect of the lipid nature, but the glyceride showed higher BA.

### Example 4: Effect of ratio lipid to drug.

NLC suspensions were prepared of identical size (appr. 200 nm) containing the same lipid content but different percentages of drug. They were 15% and 30% TU in the lipid particle mass of the suspensions (example: suspension contained 90% surfactant solution and 10% particles, the particles themselves were composed of 42.5% solid lipid, 42.5% oil and 15% TU, which corresponds to 4.25%, 4.25% and 1.5% related to the total suspension).

The compositions of the NLC suspensions were:
1.5% TU (w/w) NLC suspension:

| | |
|---|---|
| solid lipid | 4.25% |
| oleic aicd | 4.25% |
| TU | 1.5% |
| Tween 80 | 2.0 % |
| water up to | 100.0% |

3.0% TU (w/w) NLC suspension:

| | |
|---|---|
| solid lipid | 3.5% |
| oleic aicd | 3.5% |
| TU | 3.0% |
| Tween 80 | 2.0% |
| water up to | 100.0% |

267mg of the 1.5% TU NLC suspension and 133.5mg of the 3.0% TU NLC suspension were administered orally to rats. The BA of the NLC suspension of 1.5% TU with the higher lipid to drug ratio was 12,933 units, the BA with the NLC suspension of 3.0% with the lower lipid to drug ratio was 13,950 units. The BA of the NLC with higher lipid:drug ratio (1.5% TU) was surprisingly not higher. This is in contrast to the state of the art with oral TU by now.

When having a lipid:drug ratio 70:30 (3.0% TU), but increasing the size from 200 nm to 600 nm lead to a decrease in BA. However all BAs were still superior to Andriol Testocaps^{®} (Fig. 4).

### Example 5: BA of nanonized T and TU without the presence of lipid

0.32 % T was suspended in 0.2 % sodium dodecyl sulphate (SDS) surfactant solution and homogenized to yield a mean PCS particle size of 864nm. The same procedure was performed with TU. The suspension contained 1 % TU, 0.2% SDS and water up to 100% (w/w). After homogenization a mean PCS size of 474 nm was obtained.

For the in vivo study 400 mg of TU suspension, and 800mg of T suspension were administered orally to rats. The BAs were 6,616 units and 4,312 units, respectively.

### Example 6: BA of nanonized TU below 100nm

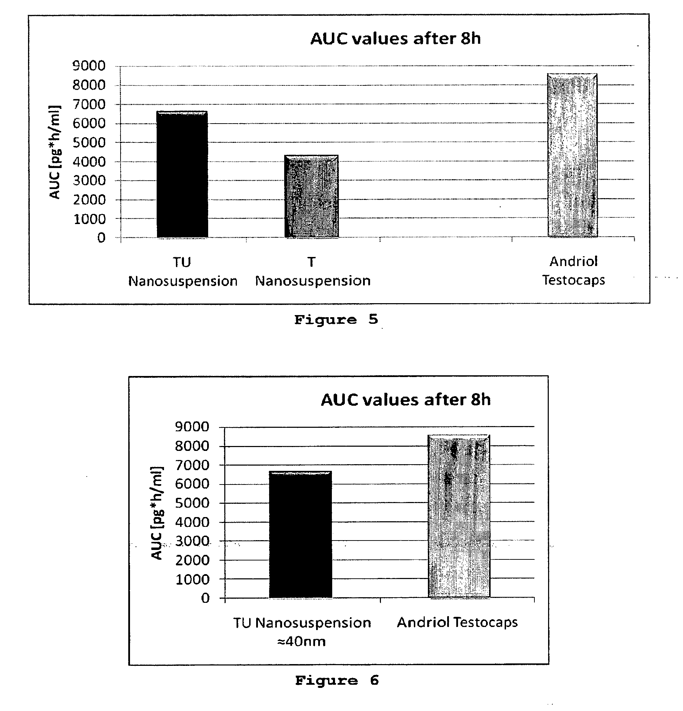

A TU suspension was prepared containing 1% TU, 1% Tween 80 and water up to 100%. This TU suspension was homogenized at elevated temperature to increase cavitation. The resulting particle size was 40 nm. 400mg of this suspension (equivalent to 4mg TU) was administered orally to rats. The BA was 6,667 units, compared to 8,542 units of the marketed product Andriol Testocaps^{®} (Fig. 6).

## Claims

1. A particulate formulation in the solid state at room temperature comprising testosterone (T) or a testosterone derivative, in particular a testosterone ester such as testosterone undecanoate (TU), with a mean particle size below 1,000 nm, which comprises said particles of testosterone or testosterone derivatives a) dispersed in a liquid outer phase and being stabilized with one or more surfactant(s) and/or one or more polymer(s) without any lipid being present or b) in a mixture with an oil and a lipid, the oil being liquid at room temperature of 20 °C and the lipid being solid at room temperature of 20° C.

2. The formulation according to claim 1, wherein the particles are dispersed in a liquid outer phase and being stabilized with one or more surfactant(s), (such as Tween 80), and/or one or more polymer(s) (such as Poloxamer, polyvinyl alcohol, polyvinyl pyrrolidone, chitosan HCl, celluloseesters).

3. The formulation according to any of claims 1 to 2, wherein the mean particle size is below 600 nm, preferably below 200 nm and more preferred below 100 nm, being most preferred at about 40 to 50 nm.

4. The formulation according to any of claims 1 to 3, wherein the particles are incorporated into tablets, buccal tablets, pellets, oral capsules, films, matrices of dermal, transdermal and mucosal patches, or in gels or creams.

5. The formulation according to any of claims 1 to 4, being composed of 1% to 50% lipid phase in 2% to 10% surfactant solution (such as Tween 80 solution), wherein the lipid phase comprises 35% stearic acid triglyceride (Dynasan 118), 35% oleic acid and 30% TU (all % are by weight) and preferably has a size of 150 to 200 nm, or below.

6. The formulation according to any of claims 1 to 4, being composed of 1% to 50% lipid phase in 1% to 5% surfactant solution (such as Tween 80 solution), wherein the lipid phase comprises 35% stearic acid triglyceride (Dynasan 118), 35% oleic acid and 30% TU (all % are weight) and preferably has a size of about 600 nm, or below.

7. The formulation according to any of claims 1 to 4, being composed of 1 to 50% lipid phase in 2% to 10% surfactant solution, such as Tween 80 solution, wherein the lipid phase comprises 42,5% stearic acid triglyceride (Dynasan 118), 42,5% oleic acid and 15% TU (all % are by weight) and preferably has a size of 150 to 200 nm, or below.

8. The formulation according to any of claims 1 to 4, being composed of 1% to 50% lipid phase in 2% to 10% surfactant solution, such as Tween 80 solution, wherein the lipid phase comprises 42,5% stearic acid, 42,5% oleic acid and 15% TU (all % are by weight) and preferably has a size of 150 to 200 nm, or below.

9. The formulation according to any of claims 1 to 4, being composed of Testosterone 0,5% in 0.2% sodium dodecyl sulphate (SDS) solution (all % are by weight) and preferably has a size of 800 nm, or below.

10. The formulation according to any of claims 1 to 4, being composed of Testosterone undecanoate 1% in 0.2% SDS solution (all % are by weight) and preferably has a size of 400 to 500 nm, or below.

11. The formulation according to any of claims 1 to 4, being composed of Testosterone undecanoate 1% in 1.0% surfactant solution, such as Tween 80 solution (all % are by weight) and preferably has a size of 40 to 50 nm, or below.

12. A method of prepararing a formulation according to any of claims 1 to 11 by dispersing the drug or the drug-lipid mixture in solution of one or more surfactant(s) and/or one or more polymer(s) and reducing it in size by high pressure homogenization (such as a piston-gap homogenizer), typically between 500 bar and 1,500 bar pressure up to 20 homogenization cycles.

13. Use of a formulation according to any of claims 1 to 11 for the preparation of a medicament, in particular in form of tablets, buccal tablets, pellets, oral capsules, films, matrices of dermal, transdermal and mucosal patches, or in gels or creams, or when the particles are dispersed in a liquid outer phase (such as an aqueous surfactant solution) as spray or as dispersion for nebulisation.

## Patentansprüche

1. Teilchenformulierung, die bei Raumtemperatur im festen Zustand vorliegt, Testosteron (T) oder ein Testosteronderivat, insbesondere einen Testosteronester wie Testosteronundecanoat (TU) umfasst, eine mittlere Teilchengröße unter 1000 nm aufweist und die Teilchen aus Testosteron oder Testosteronderivaten a) dispergiert in einer äußeren flüssigen Phase und stabilisiert mit einem oder mehreren Tensid(en) und/oder einem oder mehreren Polymer(en), wobei kein Lipid vorhanden ist, oder b) in einer Mischung mit einem Öl und einem Lipid umfasst, wobei das Öl bei Raumtemperatur von 20°C flüssig ist und das Lipid bei Raumtemperatur von 20°C fest ist.

2. Formulierung nach Anspruch 1, bei der die Teilchen in einer flüssigen äußeren Phase dispergiert und mit einem oder mehreren Tensid(en) (wie Tween 80) und/oder einem oder mehreren Polymer(en) (wie Poloxamer, Polyvinylalkohol, Polyvinylpyrrolidon, Chitosan HCl, Cleluloseestern) stabilisiert sind.

3. Formulierung nach einem der Ansprüche 1 bis 2, bei der die mittlere Teilchengröße unter 600 nm, vorzugsweise unter 200 nm und bevorzugter unter 100 nm liegt, wobei etwa 40 bis 50 nm am meisten bevorzugt ist.

4. Formulierung nach einem der Ansprüche 1 bis 3, bei der die Teilchen in Tabletten, Backentabletten, Pellets, Oralkapseln, Filme, Matrizen von Dermal-, Transdermal-und Schleimhautpflastern oder in Gele oder Cremes eingearbeitet sind.

5. Formulierung nach einem der Ansprüche 1 bis 4, die aus 1% bis 50% Lipidphase in 2% bis 10% Tensidlösung (wie eine Tween 80 Lösung) zusammengesetzt ist, wobei die Lipidphase 35% Stearinsäuretriglycerid (Dynasan 118), 35% Ölsäure und 30% TU (alle % sind auf das Gewicht bezogen) umfasst, und vorzugsweise eine Größe von 150 bis 200 nm oder weniger aufweist.

6. Formulierung nach einem der Ansprüche 1 bis 4, die aus 1% bis 50% Lipidphase in 1% bis 5% Tensidlösung (wie eine Tween 80 Lösung) zusammengesetzt ist, wobei die Lipidphase 35% Stearinsäuretriglycerid (Dynasan 118), 35% Ölsäure und 30% TU (alle % sind auf das Gewicht bezogen) umfasst, und vorzugsweise eine Größe von etwa 600 nm oder weniger aufweist.

7. Formulierung nach einem der Ansprüche 1 bis 4, die aus 1% bis 50% Lipidphase in 2% bis 10% Tensidlösung, wie eine Tween 80 Lösung, zusammengesetzt ist, wobei die Lipidphase 42,5% Stearinsäuretriglycerid (Dynasan 118), 42,5% Ölsäure und 15% TU (alle % sind auf das Gewicht bezogen) umfasst, und vorzugsweise eine Größe von 150 bis 200 nm oder weniger aufweist.

8. Formulierung nach einem der Ansprüche 1 bis 4, die aus 1% bis 50% Lipidphase in 2% bis 10% Tensidlösung, wie eine Tween 80 Lösung, zusammengesetzt ist, wobei die Lipidphase 42,5% Stearinsäure, 42,5% Ölsäure und 15% TU (alle % sind auf das Gewicht bezogen) umfasst, und vorzugsweise eine Größe von 150 bis 200 nm oder weniger aufweist.

9. Formulierung nach einem der Ansprüche 1 bis 4, die aus Testosteron 0,5% in 0,2% Natriumdocecylsulfat(SDS)lösung (alle % sind auf das Gewicht bezogen) zusammengesetzt ist, und vorzugsweise eine Größe von 800 nm oder weniger aufweist.

10. Formulierung nach einem der Ansprüche 1 bis 4, die aus Testosteronundecanoat 1% in 0,2% SDS-Lösung (alle % sind auf das Gewicht bezogen) zusammengesetzt ist, und vorzugsweise eine Größe von 400 bis 500 nm oder weniger aufweist.

11. Formulierung nach einem der Ansprüche 1 bis 4, die aus Testosteronundecanoat 1% in 1,0% Tensidlösung, wie eine Tween 80 Lösung, (alle % sind auf das Gewicht bezogen) zusammengesetzt ist, und vorzugsweise eine Größe von 40 bis 50 nm oder weniger aufweist.

12. Verfahren zur Herstellung einer Formulierung gemäß einem der Ansprüche 1 bis 11 durch Dispergieren des Wirkstoffs oder der Wirkstoff-Lipid-Mischung in einer Lösung von einem oder mehreren Tensid(en) und/oder einem oder mehreren Polymer(en) und Reduzieren der Größe durch Hochdruckhomogenisation (wie mit einem Kolben-Spalt-Homogenisator) typischerweise bei 500 bis 1500 bar Druck und bis zu 20 Homogenisierungszyklen.

13. Verwendung einer Formulierung gemäß einem der Ansprüche 1 bis 11 zur Herstellung eines Medikaments, insbesondere in Form von Tabletten, Backentabletten, Pellets, Oralkapseln, Filmen, Matrizen von Dermal-, Transdermal- und Schleimhautpflastern, oder in Gelen oder Cremes, oder, wenn die Teilchen in einer äußeren flüssigen Phase dispergiert sind (wie einer wässrigen Tensidlösung), als Spray oder als Dispersion zur Vernebelung.

## Revendications

1. Une formulation particulaire à l'état solide à température ambiante composée de testostérone (T) ou d'un dérivé de testostérone, plus particulièrement un ester de testostérone tel que l'undécanoate de testostérone (UT), ayant une dimension particulaire moyenne inférieure à 1 000 nm, dont lesdites particules de testostérone ou de dérivé de testostérone sont a) dispersées dans une phase externe liquide et stabilisées à l'aide d'un ou plusieurs tensioactifs et/ou d'un ou plusieurs polymères en l'absence de tout lipide ou b) dans un mélange avec une huile et un lipide, l'huile étant liquide à température ambiante de 20°C et le lipide étant solide à température ambiante de 20°C.

2. La formulation décrite à la revendication 1, où les particules sont dispersées dans une phase externe liquide et stabilisées à l'aide d'un ou plusieurs tensioactifs (par ex. Tween 80) et/ou d'un ou plusieurs polymères (par ex. poloxamère, alcool polyvinylique, polyvinylpyrrolidone, mélange chlorure d'hydrogène-chitosane, esters de cellulose).

3. La formulation décrite aux revendications 1 et 2, où la dimension particulaire moyenne est inférieure à 600 nm, de préférence inférieure à 200 nm, préférablement inférieure à 100 nm et idéalement d'environ 40 à 50 nm.

4. La formulation décrite aux revendications 1 à 3, où les particules sont incorporées à des comprimés, des comprimés buccaux, des pastilles, des capsules à prise par voie orale, des films, des matrices de patchs cutanés, transdermiques ou muqueux, des gels ou des crèmes.

5. La formulation décrite aux revendications 1 à 4, composée de 1 % à 50 % de phase lipidique dans 2 % à 10 % de solution tensioactive (par ex. Tween 80 en solution), où la phase lipidique se compose à 35 % de triglycéride d'acide stéarique (Dynasan 118), à 35 % d'acide oléique et à 30 % d'undécanoate de testostérone (tous les pourcentages précités étant en poids) et a de préférence une dimension particulière moyenne de 150 à 200 nm ou moins.

6. La formulation décrite aux revendications 1 à 4, composée de 1 % à 50 % de phase lipidique dans 1 % à 5 % de solution tensioactive (par ex. Tween 80 en solution), où la phase lipidique se compose à 35 % de triglycéride d'acide stéarique (Dynasan 118), à 35 % d'acide oléique et à 30 % d'undécanoate de testostérone (tous les pourcentages précités étant en poids) et a de préférence une dimension particulière moyenne de 600 nm ou moins.

7. La formulation décrite aux revendications 1 à 4, composée de 1 % à 50 % de phase lipidique dans 2 % à 10 % de solution tensioactive (par ex. Tween 80 en solution), où la phase lipidique se compose à 42,5 % de triglycéride d'acide stéarique (Dynasan 118), à 42,5 % d'acide oléique et à 15 % d'undécanoate de testostérone (tous les pourcentages précités étant en poids) et a de préférence une dimension particulière moyenne de 150 à 200 nm ou moins.

8. La formulation décrite aux revendications 1 à 4, composée de 1 % à 50 % de phase lipidique dans 2 % à 10 % de solution tensioactive (par ex. Tween 80 en solution), où la phase lipidique se compose à 42,5 % d'acide stéarique, à 42,5 % d'acide oléique et à 15 % d'undécanoate de testostérone (tous les pourcentages précités étant en poids) et a de préférence une dimension particulière moyenne de 150 à 200 nm ou moins.

9. La formulation décrite aux revendications 1 à 4, composée à 0,5 % de testostérone dans une solution de dodécylsulfate de sodium (SDS) à 0,2 % (tous les pourcentages précités étant en poids) et ayant de préférence une dimension particulière moyenne de 800 nm ou moins.

10. La formulation décrite aux revendications 1 à 4, composée à 1 % d'undécanoate de testostérone dans une solution de dodécylsulfate de sodium (SDS) à 0,2 % (tous les pourcentages précités étant en poids) et ayant de préférence une dimension particulière moyenne de 400 à 500 nm ou moins.

11. La formulation décrite aux revendications 1 à 4, composée à 1 % d'undécanoate de testostérone dans une solution tensioactive à 1,0 % (par ex. Tween 80 en solution) (tous les pourcentages précités étant en poids) et ayant de préférence une dimension particulière moyenne de 40 à 50 nm ou moins.

12. Un procédé de préparation de la formulation décrite dans n'importe laquelle des revendications 1 à 11 en dispersant le médicament ou le mélange médicament-lipide dans une solution d'un ou plusieurs tensioactifs et/ou d'un ou plusieurs polymères et en la faisant réduire par homogénéisation à haute pression (par ex. dans un homogénéisateur à piston), généralement à une pression comprise entre 500 et 1500 bars et pendant un maximum de 20 cycles d'homogénéisation.

13. L'emploi de la formulation décrite dans n'importe laquelle des revendications 1 à 11 pour préparer un médicament, plus particulièrement sous la forme de comprimés, de comprimés buccaux, de pastilles, de capsules à prise par voie orale, de films, de matrices de patchs cutanés, transdermiques ou muqueux, de gels ou de crèmes, ou lorsque les particules sont dispersées dans une phase externe liquide (par ex. une solution tensioactive aqueuse) sous la forme d'un spray ou d'une dispersion pour nébulisation.
